# EUROPÄISCHE PATENTANMELDUNG

(11) **EP 0 619 479 A1**
(43) Veröffentlichungstag der Anmeldung: **12.10.1994**
(21) Anmeldenummer: 94103855.6
(22) Anmeldetag: 14.03.1994
(51) Int. Cl.: G01N 1/20

(54) **Rohrleitungselement zur Probenentnahme**

(30) Priorität: 07.04.1993 DE 9305277 U
(71) Anmelder: G.U.T.E. GmbH GESELLSCHAFT FÜR UMWELT, TECHNIK UND ENTSORGUNG, D-35428 Langgöns-Espa (DE)
(72) Erfinder: Zander, Dietmar, D-35428 Langgöns-Espa (DE)
(74) Vertreter: Missling, Arne, Dipl.-Ing.

(57) **Zusammenfassung**

Die Anmeldung bezieht sich auf eine Probenentnahmevorrichtung mit einem Rohrleitungselement (1), welches in einer Wandung (10) eine Öffnung (11) aufweist, an welcher eine durchstechbare Membran (2) gelagert ist.

## Beschreibung

Die Erfindung bezieht sich auf ein Rohrleitungselement sowie auf eine Probenentnahmevorrichtung.

Es sind unterschiedlichste Anwendungsfälle bekannt, bei welchen eine Probe einer Flüssigkeit aus einem Rohrleitungselement entnommen werden muß. So ist es beispielsweise möglich, zur Untersuchung der Güte von Trinkwasser Proben zu ziehen, welche hinsichtlich ihrer physikalischen, chemischen und/oder mikrobiologischen Eigenschaften untersucht werden müssen. Bei derartigen Probeentnahmen ist es erforderlich, eine repräsentative Probe zu ziehen, d. h. eine Probe aus einem durchströmten Rohrleitungsstück zu entnehmen. Weiterhin muß die Probenentnahme so erfolgen, daß Kontaminationen oder Verschmutzungen, welche durch die Probenentnahme selbst hervorgerufen werden, vermieden werden können.

Bei den bekannten Vorgehensweisen bei der Entnahme von Trinkwasserproben aus Rohrleitungssystemen erfolgt die Probenentnahme an einem üblichen Wasserauslaß, beispielsweise einem Seitenstrang oder einem Wasserhahn. Dieser Wasserauslaß muß vor der Probenentnahme vollständig von stehendem Wasser leergespült werden. Bereits das Leerspülen bereitet Probleme, da nicht feststellbar ist, wann ein ausreichend leergespülter Zustand erreicht ist. Es wird dann der Wasserauslaß mit geeigneten Mitteln desinfiziert oder gereinigt, beispielsweise mittels eines Bunsenbrenners abgeflämmt, um Keime bzw. einen vorhandenen Biofilm abzutöten. Nach diesem Vorgang wird der Wasserauslaß erneut geöffnet, um Wasser in ein steriles Gefäß abzufüllen. Diese Vorgehensweise gewährleistet jedoch keine sterile Probenentnahme, da zum einen die Reinigung des Wasserauslasses nicht in zuverlässiger Weise überprüft werden kann und zum anderen auch das Ausfüllen des Wassers in ein steriles Gefäß zu Verunreinigungen bzw. Kontaminationen führen kann.

Der Erfindung liegt die Aufgabe zugrunde, ein Rohrleitungselement und eine Probenentnahmevorrichtung zu schaffen, welche unter Vermeidung der Nachteile des Standes der Technik eine sterile Probenentnahme aus einem Rohrleitungssystem ermöglichen.

Erfindungsgemäß wird die Aufgabe hinsichtlich des Rohrleitungselements dadurch gelöst, daß an einer Öffnung der Wandung des Rohrleitungselements eine dicht eingebaute, durchstechbare Membrane angeordnet ist.

Die erfindungsgemäße Ausgestaltung des Rohrleitungselements zeichnet sich durch eine Reihe erheblicher Vorteile aus. Da die Membrane in einer Öffnung der Wandung des Rohrleitungselements eingebaut ist, ergeben sich keine Totzonen mit stehender Flüssigkeit, in welcher sich Verunreinigungen oder ähnliches ansammeln könnten. Hierdurch ist gewährleistet, daß bei der Probenentnahme eine repräsentative Probe entnommen werden kann. Weiterhin entfallen die aus dem Stand der Technik bekannten Vorarbeiten, nämlich das Freispülen und Reinigen/Desinfizieren der Entnahmestelle. Die Unterseite der Membrane ist vielmehr stets von der Wasserströmung berührt, so daß diese nicht gereinigt werden braucht. Die Oberseite der durchstechbaren Membrane kann in einfachster Weise, beispielsweise durch Aufsprühen eines Desinfektionsmittels gereinigt werden.

Bevorzugterweise ist die Membrane in einem von dem Rohrleitungselement abzweigenden Rohrstutzen aufgenommen. Diese Aufnahme der Membrane erleichtert zum einen den Einbau, zum anderen ist die Membrane vor Beschädigungen geschützt. Zusätzlich ist sichergestellt, daß die Membrane drucksicher eingebaut werden kann, so daß Undichtigkeiten des Rohrleitungssystems ausgeschlossen werden können.

Die Membrane ist bevorzugterweise mit ihrer innenliegenden Oberfläche angrenzend an den freien Strömungsquerschnitt des Rohrleitungselements angeordnet. Hierdurch wird vermieden, daß sich im Bereich der Membrane Totzonen oder ähnliches bilden, in welchen Verunreinigungen auftreten könnten.

Die Membrane, welche bevorzugterweise aus einem durchstechbaren Kunststoffmaterial gefertigt ist, ist in günstiger Weise vorgespannt in dem Rohrstutzen gelagert. Hierdurch ist es möglich, die Membrane mittels einer Probenentnahme-Kanüle zu durchstechen, um eine Probe zu ziehen. Nach dem Entfernen der Kanüle schließt sich die Öffnung der Membrane wieder dicht, so daß das Austreten von Wasser bzw. das Eintreten von Verunreinigungen vermieden wird.

Die Lagerung der Membrane erfolgt in günstiger Weise mittels eines in dem Rohrstutzen eingeschraubten Pressringes. Durch diese Ausgestaltung ist es möglich, zum einen die Membrane in ausreichend dichter Weise einzubauen und zum anderen die Membrane bei Bedarf auszutauschen.

Der Rohrstutzen ist zur Vermeidung von Beschädigungen der Membrane und um zusätzliche Verschmutzungen zu vermeiden, mittels einer Verschlußkappe abdichtbar. Die Verschlußkappe kann auch absperrbar oder nur mit einem Spezialwerkzeug zu öffnen sein, um Manipulationen an der Membrane auszuschliessen.

Erfindungsgemäß ist die Kanüle steril und mit einem ebenfalls sterilen Probenentnahmebehälter, beispielsweise einer Flasche oder einem Beutel verbunden. Diese Verbindung kann mittels eines Schlauches erfolgen. Die Kanüle ist bevorzugterweise in Form einer Löffelschliffkanüle oder einer HUBER-Kanüle ausgebildet. Durch diese Form der Kanülen wird ein Ausstanzen von Membranenmaterial beim Einstechen der Kanüle verhindert, so daß die Membrane nach dem Herausziehen der Kanüle wieder dicht schließt.

Erfindungsgemäß ist es somit möglich, an jeder beliebigen Stelle eines Rohrsystems in einfachster Weise sterile Proben zu entnehmen.

Im folgenden wird die Erfindung anhand eines Ausführungsbeispiels in Verbindung mit der Zeichnung beschrieben. Dabei zeigt:
- Fig. 1: eine Schnitt-Seitenansicht eines Ausführungsbeispiels des erfindungsgemäßen Rohrleitungselements und
- Fig. 2: eine schematische Darstellung der erfindungsgemäßen Kanüle und des Probenentnahmegefäßes.

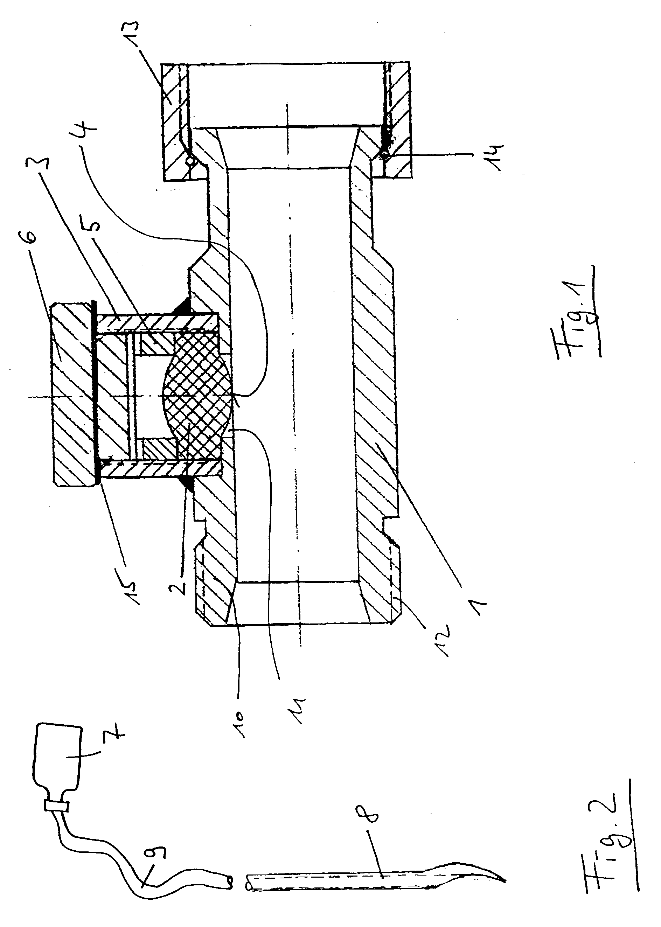

Die Fig. 1 zeigt ein Rohrleitungselement 1, welches in üblicher Weise mit Anschlußelementen versehen ist, beispielsweise einem Gewinde 12, einer Überwurfmutter 13 sowie einem Federring 14 zur Befestigung der losen Übermutter 13 mit dem Element 1. Somit kann das Rohrleitungselement 1 wie ein üblicher Rohrfitting in eine Rohrleitung eingebaut werden.

Das Rohrleitungselements 1 weist bevorzugterweise einen kreisrunden Querschnitt auf und ist mit einer Wandung 10 versehen, in welche eine Öffnung 11 eingebracht ist, beispielsweise in Form einer Bohrung. Die Öffnung 11 wird umschlossen von einem Rohrstutzen 3, welcher mit dem Rohrleitungselement fest verbunden ist, beispielsweise durch verschweißen. Der Rohrstutzen 3 ist mit einem Innengewinde versehen, in welches ein Pressring 5 einschraubbar ist. Auf das freie Ende des Rohrstutzens 3 kann eine Verschlußkappe 6 lösbar aufgeschraubt werden. Zu deren Abdichtung kann eine zusätzliche Dichtung 15 vorgesehen sein.

Mit Hilfe des Pressrings 5 ist in dem Rohrstutzen 3 eine scheibenförmige Membrane 2 eingespannt. Die innenliegende Oberfläche 4 der Membrane 2 ist so angeordnet, daß sie an den freien Strömungsquerschnitt des Rohrleitungselements 1 angrenzt.

Nach Abnahme der Verschlußkappe 6 und Desinfizieren der Aussenseite der Membrane 2 kann diese mittels einer Kanüle 8 durchstoßen werden, um aus dem Innenraum des Rohrleitungselements 1 eine flüssige Probe abzuziehen. Zu diesem Zwecke ist die Kanüle 8 mit einem Schlauch 9 verbunden, welcher in einen Probenentnahmebehälter 7 mündet. Die Kanüle 8 ist bevorzugterweise steril, so wie dies aus dem Medizintechnikgebiet bekannt ist. In gleicher Weise ist der Schlauch 9 und der Probenentnahmebehälter 7 steril. Nach der Probenentnahme kann der Schlauch 9 abgeklemmt werden, um eine Kontamination des Inhalts des Probenentnahmebehälters 7 zu vermeiden.

Die Erfindung ist nicht auf das gezeigte Ausführungsbeispiel beschränkt, vielmehr ergeben sich für den Fachmann im Rahmen der Erfindung vielfältige Abwandlungs- und Modifikationsmöglichkeiten.

## Patentansprüche

1. Rohrleitungselement, dadurch gekennzeichnet, daß an einer Öffnung (11) der Wandung (10) das Rohrleitungselements (1) dicht eine durchstechbare Membrane (2) angeordnet ist.

2. Rohrleitungselement nach Anspruch 1, dadurch gekennzeichnet, daß die Membrane (2) in einem von dem Rohrleitungselement (1) abzweigenden Rohrstutzen (3) aufgenommen ist.

3. Rohrleitungselement nach Anspruch 1 oder 2, dadurch gekennzeichnet, daß die Membrane (2) mit ihrer innenliegenden Oberfläche (4) angrenzend an den freien Strömungsquerschnitt des Rohrleitungselements (1) angeordnet ist.

4. Rohrleitungselement nach Anspruch 2 oder 3, dadurch gekennzeichnet, daß die Membrane (2) vorgespannt in dem Rohrstutzen (3) gelagert ist.

5. Rohrleitungselement nach Anspruch 4, dadurch gekennzeichnet, daß die Membrane (2) mittels eines in den Rohrstutzen (3) einschraubbaren Pressrings (5) eingespannt ist.

6. Rohrleitungselement nach einem der Ansprüche 2 bis 5, dadurch gekennzeichnet, daß Rohrstutzen (3) mittels einer Verschlußkappe (6) abdeckbar ist.

7. Rohrleitungselement nach einem der Ansprüche 1 bis 6, dadurch gekennzeichnet, daß die Membrane (2) aus einem durchstechbaren Kunststoffmaterial gefertigt ist, die sich nach Entfernen der Kanüle wieder druckdicht verschließt.

8. Probenentnahmevorrichtung, gekennzeichnet durch das Rohrleitungselement (1) nach einem der Ansprüche 1 bis 7 und eine mit einem Probenentnahmebehälter (7) verbundene Kanüle (8).

9. Probenentnahmevorrichtung nach Anspruch 8, dadurch gekennzeichnet, daß die Kanüle (8) steril ist.

10. Probenentnahmevorrichtung nach einem der Ansprüche 8 oder 9, dadurch gekennzeichnet, daß die Kanüle (8) mit dem Probenentnahmebehälter (7) über einen Schlauch (9) verbunden ist.
